# EUROPEAN PATENT APPLICATION

(11) **EP 4 202 032 A1**
(43) Date of publication of application: **28.06.2023**
(21) Application number: 22213310.0
(22) Date of filing: 14.12.2022
(51) Int. Cl.: C12M 1/12, C12M 1/00, C12M 1/02, C12M 1/34

(54) **IMPROVED CELL CULTURE METHOD**

(30) Priority: 21.12.2021 WO PCT/US2021/064584
(71) Applicant: Bayer AG, 51373 Leverkusen (DE)
(72) Inventor: Krasenbrink, Jürgen, 51063 Köln (DE); Zhang, Yongchun, Walnut Creek, 945951601 (US)
(74) Representative: BIP Patents

(57) **Abstract**

What is describe herein relates to a method for improving a cell culture comprising
a) heating a cell culture process fluid using a single-use dense membrane hollow fiber module wherein said single-use dense membrane hollow fiber module comprises a plurality of dense membranes arranged as hollow fibers in a housing
and/or
b) wherein the cell culture has at least one cell culture chamber and at least the bottom or at least the top of said cell culture chamber is covered with an insulation.

## Description

In recent years cell and gene therapy has been considered as a new way for treatment especially of diseases which are so far difficult to treat. Whereas in general cell therapy aims to treat diseases by restoring or altering certain sets of cells, gene therapy aims at treating diseases by replacing, activating/deactivating or introducing genes into cells. Moreover, some therapies are considered both cell and gene therapies. These therapies work by altering genes in specific types of cells and inserting them into the body. Thus especially for cell and gene therapy as well as cell therapy applications cell culture of the relevant cell types is crucial. Hence a reliable, consistent cell culture process is required.

### SUMMARY OF THE INVENTION

In a first aspect what is described herein relates to a method for improving a cell culture comprising
a) heating a cell culture process fluid using a single-use dense membrane hollow fiber module (1) wherein said single-use dense membrane hollow fiber module comprises a plurality of dense membranes arranged as hollow fibers (2) in a housing (3)
   and/or
b) wherein the cell culture has at least one cell culture chamber and at least the bottom or at least the top of said cell culture chamber (7) is covered with an insulation.

In a second aspect what is described herein relates to the use of a dense membrane hollow fiber module as single use heat exchanger.

### DESCRIPTION OF THE DRAWINGS

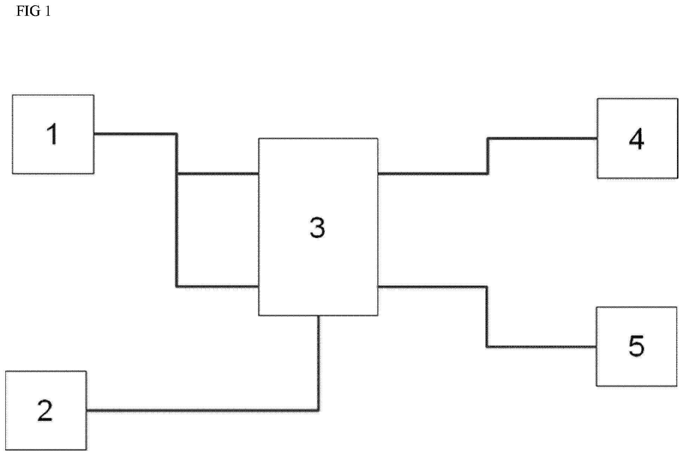

FIG. 1 depicts an exemplary arrangement in which a dense membrane hollow fiber module was used as single use heat exchanger. In detail a heating fluid (also termed heating solution) was provided in reservoir (4) whereas a process fluid to be warmed e.g. a cell culture medium was provided in reservoir (2). During operation the heating solution entered the hollow fibers of a single-use dense membrane module (3) flowed through the hollow-fibers and exited the single-use dense membrane module (3) towards the reservoir (5) while the process fluid to be warmed flowed over the outside of the hollow fibers (i.e. the shell side) and exited the single-use dense membrane module in a sterile fashion towards the process (1). Hence the process fluid, which was warmed in the single-use dense membrane hollow fiber module (3), was used outside of the single-use dense membrane hollow fiber module itself (3).

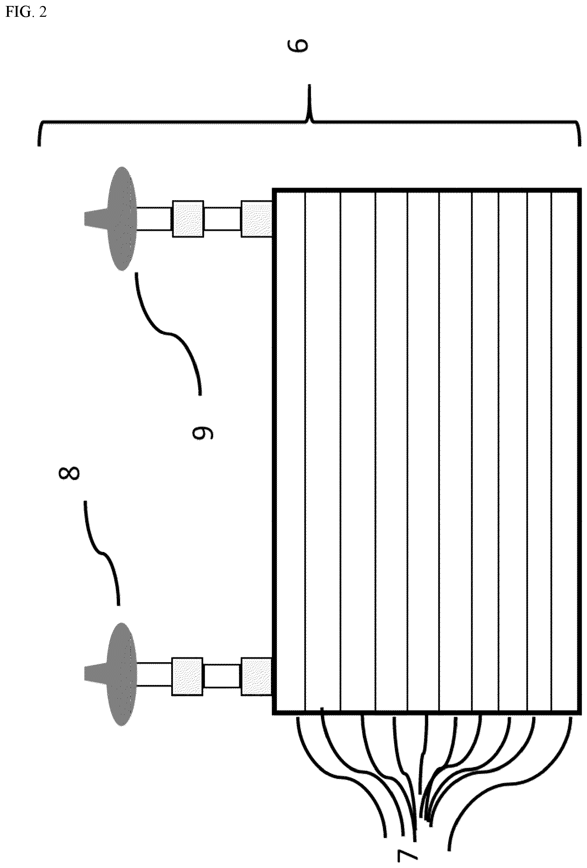

FIG. 2 depicts an example of a stacked cell culture (6), comprising in this example 10 cell culture chambers (7) as well as an inlet/outlet for fluid (8) and an inlet/outlet for gas (9).

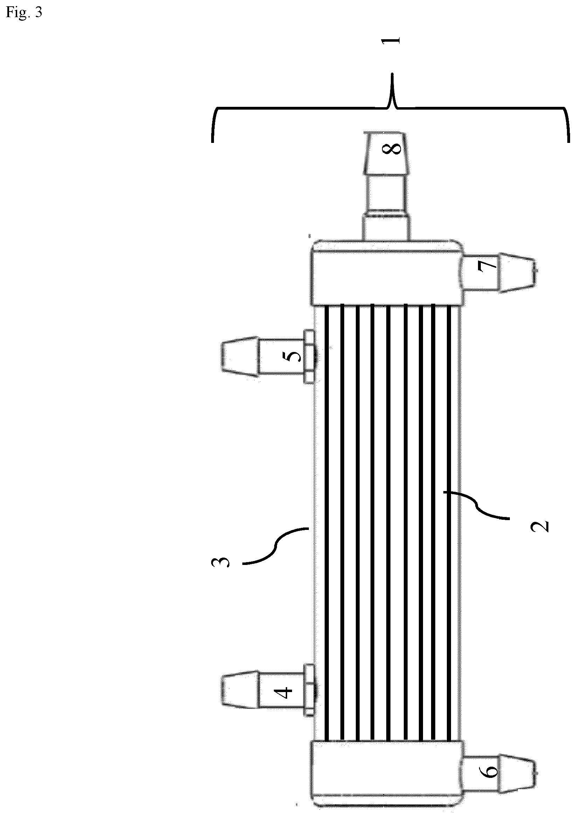

FIG 3 depicts a dense membrane hollow fiber module (1) as it was used in the set up of FIG: 1 as single use dense membrane hollow fiber module. The dense membrane hollow fiber module (1) comprised a plurality of dense membranes formed as hollow fibers (2) packaged into a housing (3). In this example the heating fluid entered the dense membrane hollow fiber module (1) at inlet (6) and exited at outlet (7) whereas a process fluid to be warmed entered the dense membrane hollow fiber module (1) at inlet (8) and exited at outlets (4) and (5) to be employed outside of the single-use dense membrane hollow fiber module itself to improve a cell culture.

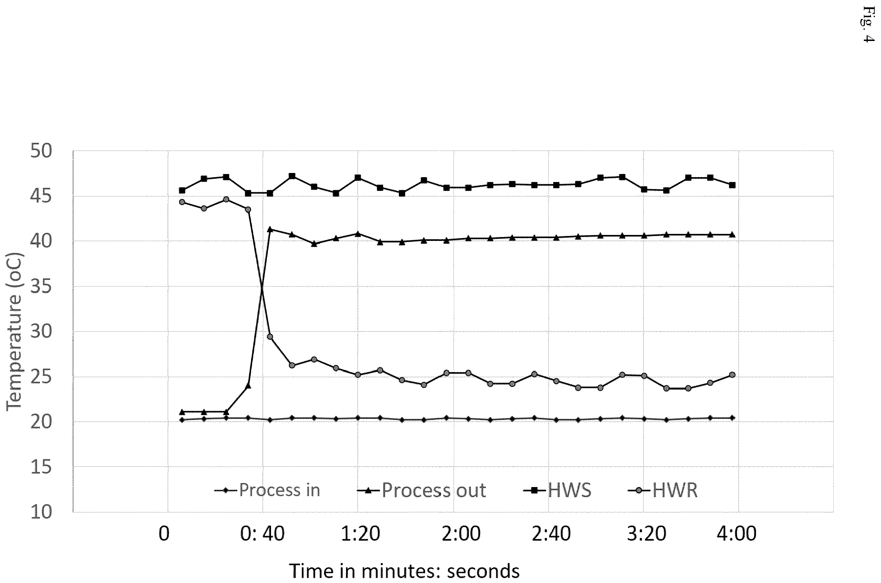

FIG. 4 demonstrates that it is possible to heat a cell culture process fluid using a dense membrane hollow fiber module, e.g. a single-use dense membrane hollow fiber module, as depicted in FIG. 3 using the process as depicted in FIG. 1 very quickly and efficiently. The abbreviation HWS stands for "heating water supply" and the abbreviation HWR stands for "heating water return". It is shown that the "heating water supply" i.e. the heating fluid constantly entered the dense membrane hollow fiber module with a temperature of around 45° C through-out the experiment. Moreover, also the process fluid to be warmed here termed "process in" entered the dense membrane hollow fiber module with a constant temperature here of around 20°C. Before the pump conveying the HWS to the single use dense membrane hollow fiber module was turned on, the tubes were filled with fluid that was colder than the heating fluid. Hence there was a time delay of about 50 sec. until the temperature of the process out i.e. the warmed up process fluid reached around 40°C at the position where the temperature sensor was located. This 50 sec time delay is considered almost instantaneous. After the initial time delay the temperature of the process out i.e. warmed up process fluid which is to be employed outside of the single-use dense membrane hollow fiber module to improve a cell culture did not drop below 40°C. The heat was transferred from the heating water supply to the process fluid to be warmed as demonstrated by the loss of heat in the "heating water return" i.e., lower temperature of the heating fluid leaving the single-use dense membrane hollow fiber module.

### DEFINITIONS

As used herein the term "stacked cell culture" refers to a cell culture method wherein at least two cell culture chambers are stacked on top of each other and have a fluid connections for simultaneously filling and removing liquids such as medium. The cell culture chambers can be fastened to each other.

Cell culture stacks are described for example in US7,867,761B2. A stacked cell culture for example consist of 1, 2, 4, 5, 10 or 40 cell culture chambers stacked on top of each other when the chambers - also termed trays - are in a horizontal position. Usually all cell culture chambers of a stacked cell culture i.e. of a cell culture stack are connected via an opening at each cell culture chamber to a first channel used for gas exchange and a second opening at each cell culture to a second channel used for medium exchange. To a skilled person it is clear, that even though the stacked cell culture consisting of 1 cell culture chamber technically does not fit the above definition this size can be of great value for scale-down experiments etc. if treated like larger stacked cell cultures.

As used herein the term "cell culture chamber" refers to housing such as a vessel having a chamber in which cells can be grown. A cell culture chamber can be coated or otherwise modified to be suitable for a given cell type e.g. adherent cells. Often cell culture chambers are referred to according to their surface area e.g. a T-175 type cell culture vessel also referred to as T-175 cell culture flask has a surface area of 175 cm².

The cell culture chambers used for the method described herein can be manufactured from high quality. optically clear polystyrene.

As used herein the term "single-use" refers a material or device which is only used once i.e. it is not cleaned or sanitized for another use.

As used herein the term "dense membrane" refers to a separation layer that is characterized by having no pores that would permit a convective mass transfer of liquid through the membrane. In other words a dense membrane comprises at least one separation layer which does not permit mass transfer via bulk motion of a fluid. Dense membranes allow substances to pass through them by a process of solution and diffusion, in which the substance dissolves into the membrane and passes through it to the other side. Thus, a dense membrane is usually permeable to gas via permeation of the gas into the membrane and exit of the gas at the other side of the membrane. Examples include polymers (such as silicone), metals (such as palladium), and ceramics.

As used herein the term "hold up volume " or "hold-up volume" refers to the volume of liquid retained in a device or housing e.g.. a filter. A skilled person is aware that the hold up volume can be expressed with or without air purge. Often, a given hold-up volume is specified by the device manufacturer, if not. it can be calculated using the dimensions of the device. for cylindrical filter housing e.g. V=pi^{∗}r^2^{∗}H.

### DETAILED DESCRIPTION

What is described herein relates to a method for improving cell culture comprising
a) heating a cell culture process fluid using a single-use dense membrane hollow fiber module (1) wherein said single-use dense membrane hollow fiber module comprises a plurality of dense membranes arranged as hollow fibers (2) in a housing (3)
   and/or
b) wherein the cell culture has at least one cell culture chamber and at least the bottom or at least the top of said cell culture chamber (7) is covered with an insulation.

It was surprisingly found that already using either option resulted in an overall improved cell culture process with a higher efficiency and/or a higher consistency.

The higher efficiency is especially attributed to the fact that if a fluid such as a cell culture process fluid is heated using a single-use dense membrane hollow fiber module the residence time of said fluid within the process is reduced, since the heating is faster as with state of the art single use heat exchangers suitable for cell culture process fluids. Thus this is especially advantageous if the cell culture process fluid is not thermally stable. Moreover, due to the smaller hold up volume of the single-use dense membrane hollow fiber module compared to state of the art single use heat exchangers suitable for cell culture process fluids less cell culture process fluid is potentially discarded rendering the method more cost efficient. Furthermore as the single-use dense membrane hollow fiber module can be incorporated into the fluid path of a cell culture process fluids process steps which would otherwise be required to heat up the process fluid(s) in parallel to the fluid path can be omitted thereby reducing processing steps and also increasing the overall process efficiency.

The higher consistency on the other hand is especially due to a more reproducible and reliable temperature of the cell culture process fluid such as a cell culture medium in each cell culture chamber regardless of whether said cell culture chamber is comprised in a cell stack or not. This consistency and reproducibility also allows for the use of a scale down model where e.g. a single cell culture chamber is used to model the process in a cell culture stack with 40 cell culture chambers.

In one embodiment of the method described herein the two parts of the method are combined i.e. a single-use dense membrane hollow fiber module is used to heat a cell culture process fluid which is to be provided to a cell culture which comprises at least one cell culture chamber and at least the bottom or at least the top of said cell culture chamber is covered with an insulation. For example a cell culture medium is heated using the single-use dense membrane hollow fiber module and then provided to a cell culture stack comprising 10 cell culture chambers wherein at least the bottom or at least the top of said cell stack cell comprising 10 cell culture chambers is covered with and insulation. Thus cells grown in said cell culture stack are exposed to less than optimal medium temperatures for a minimal amount of time since the new medium is pre-heated and the insulation prevents the old medium from cooling during medium exchange. Furthermore due to the insulation all cells in the cell stack experience the same culture conditions, since the temperature profile between layers is consistent via employing the insulation as well as the pre-heated medium. Hence the combination results in more consistent cell culture which can be scaled down, and potentially leads to higher cell yields.

In a dense membrane hollow fiber module a plurality of dense membranes is formed into fibers, which are hollow on the inside i.e. inside of the fiber is a channel. These fibers are bundled in a very compact into a housing. The housing and hence the membrane module typically has an inlet and an outlet: These are usually in "communication" with the inside of all the hollow fibers (also known as tube side). Moreover, the membrane modules will have one or more ports in fluid communication to the outside of the hollow fibers termed "shell side".

To a skilled person it is clear that while the dense membrane hollow fiber module is used as single-use dense membrane hollow fiber module in the applications described herein the module does not have to be employed as single-use article.

In one embodiment of the method described herein the singe-use dense membrane hollow fiber module comprises a tube side and a shell side and during the heating of the cell culture process fluid using said single-use dense membrane hollow fiber module (1) a heating fluid is passed through the tube side of said single-use dense membrane hollow fiber module (1) while the cell culture process fluid to be warmed is passed through the shell side of said single-use dense membrane hollow fiber module (1) or vice versa.

In one embodiment of the method described herein the cell culture process fluid is sterile.

The cell culture process fluid is for example selected from the group consisting of a cell culture medium a coating fluid employed to coat a cell culture chamber before culture of adherent cells in said chamber a washing fluid or a fluid to remove the cells from the cell culture etc.

In an especially one embodiment of the method described herein the cell culture process fluid is a cell culture medium.

In one embodiment of the method described herein the single-use dense membrane hollow fiber module is a single-use dense silicone membrane hollow fiber module, i.e. the membranes which form the hollow fibers of the module are made from silicone.

In one embodiment of the method described herein the single-use dense membrane hollow fiber module has a diameter in the range of between 2 cm and 12 cm. preferably between 5 cm and 10 cm. most preferably 8.9 cm.

In one embodiment of the method described herein the single-use dense membrane hollow fiber module has an area in the range of between 1 m² and 4 m² preferably between 1.5 m² and 3 m^{2.} even more preferably of 2.1m^{2.}.

In one embodiment of the method described herein the single-use dense membrane hollow fiber module has a length in the range of between 5 cm and 20 cm. preferably between 10 cm and 17 cm. even more preferably of 14.2 cm.

In one embodiment of the method described herein the single-use dense membrane hollow fiber module comprises hollow silicone fibers knitted in a mat with central feed inlet and has a polycarbonate shell and a polyurethane potting. In one embodiment the single-use dense membrane hollow fiber module is a PDMSXA-2.1: PermSelect^{®} 2.1 m² membrane module.

Typical cells that are used in cell cultures are stem cells, CHO cells, baby hamster kidney cell, neural progenitor cells or neural cells.

In one embodiment of the method described herein the cell culture is a cell and gene therapy cell culture.

In a one embodiment of the method described herein the heating is carried out via passing the cell culture process fluid through a single-use dense membrane hollow fiber module with a hold up volume in the range of between 20 ml and 400 ml, preferably between 100 ml and 300 ml, most preferably 200 ml. In contrast. the hold-up volume of the existing single-use heat exchanger on the market is 6.0 liters.

In an embodiment of the method described herein the volume of cell culture process fluid that is heated is in the range of between 1.5 liters and 50 liters, preferably between 2 liters and 30 liters.

In an embodiment of the method described herein the heating is carried out at a flow rate of the cell culture process fluid in the range of between 0.5 1/min and 10 1/min, preferably between 0.8 1/min and 2.1/min, most preferably between 1 1/min and 4 1/min.

In an embodiment of the method described herein heating warms the cell culture process fluid from between 20-22 °C to 35-42°C.

In an embodiment of the method described herein the heating requires passing the medium one to three times through the dense membrane hollow fiber module.

In a one embodiment of the method described herein the heating only requires passing the cell culture process fluid once through the single-use dense membrane hollow fiber module.

In another one embodiment of the method described herein the heating takes place in the range of between 5 sec to 5 min, preferably between 7 sec to 4 min most preferably between 10 sec to 3 min.

Moreover it is clear to a person skilled in the art, that the single-use dense membrane hollow fiber module could also be used to heat up gasses required in the cell culture process. However, also in such a scenario the heating fluid - i.e. the fluid that heats up the gas to be warmed - would be a fluid not another gas as using a fluid as heating medium is more efficient.

In an further one embodiment of the method described herein the cell culture is a stacked cell culture and at least the top or the bottom cell culture chamber are insulated. In one embodiment both the top and the cell culture chamber of the stacked cell culture are insulated.

In an embodiment of the method described herein the insulation is made from thermoplastic material, such as expanded polystyrene.

In an embodiment of the method described herein the insulation is added prior to establishing the cell culture in a given chamber.

In an embodiment of the method described herein the insulation is added after establishing cell culture in a given chamber.

In an embodiment of the method described herein the insulation is added during establishing cell culture in a given chamber.

In a second aspect what is described herein relates to the use of a dense membrane hollow fiber module as single use heat exchanger.

### DESCRIBTION OF THE EMBODIMENTS

### EXAMPLES

### 1. Cell Culture Characterization

In order to assess how cell culture in stacked cultivation systems could be improved various parameters were tested in both stacked cell culture systems from ThermoFisher as well as Corning. In order to do so parameters such as temperature, pH, pO₂ and pCO₂ were measured after filling the individual cell culture chambers, i.e. the layers/plates of a given stacked cell culture system with 2-8°C, room temperature or warm medium or applying passive vs. active gassing. For the O₂, CO₂ and pH measurements sensor spots were used whereas for the temperature mapping a temperature logger (Ahlbom. Almemo 710 V7) with 10 dipping probes was employed. The probes were located either centrally, laterally or at different positions in each layer of a stack with 1 or 10 layers. respectively. A CO₂ incubator (Kuhner. Climo-Shaker ISF1-XC) and an aquarium pump where applicable for active gassing were used for the experiments.

It was found (cf. Table 1) that the inner temperature kinetics of the CS10 and CF 10 stacked cell culture systems were very similar.

**Table 1**

| Warm-up time in h | CS10 | CF10 | CF4 | CS1 |
|---|---|---|---|---|
| From ∼ 21° C to 37 ± 1°C | 2.9-6.2 | 2.9-6.6 | 2.1-3.3 | 0.8-0.9 |

However, it was surprisingly determined that the variability between the different layers was much higher than expected. In other words, at the start it was assumed that if at all the middle layers would only need slightly longer to reach 37°C than the outer layers. On the contrary as depicted in Table 2 the temperature in the top and bottom layers of the CS10 and CF 10 stacked cell culture systems reached the desired 37 ± 1°C within approximately 3 hours, while the middle layers only reached this temperature after approximately 6 hours and hence needed not a little longer but twice as long for equilibration. The same was true even if pre-warmed media and cells stacks were used as depicted in Table 3, i.e., if the situation of a media change is mimicked. In order to evaluate this scenario cell layers were filled with water and the respective cell stacks were incubated at 37°C. Once the water in all layers in the incubator had reached 37°C a medium exchange using pre-warmed medium and pre-warmed cell stacks was mimicked via exposing the cell stacks to room temperature for 1,2h. After this time the cell stacks were placed back into the incubator and the time it took all layers to reach 37°C was monitored. The results are depicted in Table 2 below.

**Table 2**

| Warm-up time in h | CS10 | CF10 |
|---|---|---|
| from ∼ 21° to 37 ± 1°C | 2.9-6.2 | 2.9 - 6.6 |
| from 37 ± 1°C back to 37 ± 1°C after 1,2 h at ∼ 22 °C | 1.5 -2.7 | 1.5-3.0 |

Therefore, it was unexpectedly found that the different layers were not comparable regarding their temperature profile meaning that cells cultured in middle layers, experienced longer times and less optimal temperatures than cells grown in top, bottom and adjacent layers. In other words, it took the cell culture medium in the middle layers twice as long to reach the temperature set by the incubator the cells were grown in than the cell culture medium in top and bottom and adjacent layers.

### 2. Insulation

Thus, in order to optimize cell culture conditions an insulation was devised for the top and bottom layers of a given stacked cell cultivation system since then all layers are exposed with the same surface area - i.e. the sides - to the external temperature environment e.g. of an incubator. The insulations were made from expanded polystyrene plates cut to match the top and bottom layers of the used stacked cell cultures. The results as depicted in Table 3 show that the insulation results in more homogenous temperature equilibration between all layers.

**Table 3**

| Warm-up time in h (from 21° C to 37 ± 1°C) | CS10 | CF10 | CF4 | CS1 |
|---|---|---|---|---|
| With insulation | 7.4 - 8.1 | 7.5 - 8.0 | 7.2-7.7 | 4.3 |
| Without insulation | 2.9 - 6.2 | 2.9-6.6 | 2.1- 3.3 | 0.9 |

Specifically the bottom, top and adjacent layers adapted their profiles to the middle layers.

The results are valid for warming and cooling phases i.e. the insulation results in a comparable temperature profile in all layers of a stacked cell culture system both in an incubator as well as during handling outside of an incubator e.g. during medium exchange, where all cells are exposed to temperatures less than 37° C as little and as slowly as possible. Hence insulating the top and bottom layers of a stacked cell culture system facilitates a high process temperature consistency throughout a system resulting in more consistent overall process and the possibility to create valid scale down models.

Alternatively or in addition to insulating the top and/or bottom layers of a stacked cell culture system or the top and bottom of a single cell culture vessel in scale down experiments, heating the cell culture medium seemed a promising approach to reduce the warm-up phase and hence achieve more process consistency and higher yields in stacked cell culture systems especially for cells requiring frequent media exchange.

### 3. Heating cell culture process fluid

Pre-warming of cell culture medium e.g. in a water bath is not feasible if the medium contains heat sensitive components such as Fibroblast Growth Factor 2. Moreover, commercially available systems such as the Thermo Scientific^{™} DHX^{™} heat exchanger offer a modular system for single-use applications where heat is transferred via up to five dimpled, stainless-steel plates, but this system did not fit the requirements of a stacked cell cultivation system for typical cell therapy applications since the required hold-up volume of the Thermo Scientific^{™} DHX^{™} heat exchanger is too large and the flow rates of the systems do not match. In detail based on DHX datasheet, a 4-BPCs assembly is required to heat water from 20 °C to 37 °C at 2 L/min water flow and 15 L/min 42 °C 30% propylene glycol flow, the hold-up volume of this system is 6 L, which is significant for cell therapy applications where the media batch is routinely less than 20 liter . However, it was surprisingly found that a single-use dense silicone membrane hollow fiber module could be used for efficiently heating cell culture process fluid while also fulfilling the other process requirements. In detail a PermSelect PDMSXA 2.1m² module was used to warm-up water from 20 °C to 40 °C at 2 L/min flow. The operation conditions are given in Table 4 below. From Table 4 it can be seen that in contrast to the manufacturer's instructions neither a sweep flow nor a sweep flow with vacuum or a gas flow was used. Hence, the way in which the single-use dense membrane hollow fiber module was used in the method for improving a cell culture via heating a cell culture process fluid differs from how the single-use dense membrane hollow fiber module has been used so far in the state of the art.

**Table 4 Comparison of operation conditions of the single-use dense silicone membrane hollow fiber module used in the example**

| | Manufactures instructions for use as degassing/gassing module | Operating conditions when used as heat exchanger |
|---|---|---|
| Max Continuous Operating Temperature °C | 60 | 47 |
| Max Shell Side Pressure bar | 1 @ 77°F | Not measured assumed to be < 0.0007 per datasheet |
| Max Lumen Pressure bar | 3 @ 77°F | Not measured assumed to be < 0.00137895 |
| Max TMP Shell to Lumen bar | 1 @ 77oF | < 0.00137895 |
| Max TMP Lumen to Shell bar | 3 @ 77oF | < 0.00137895 |
| Typical Liquid Flow Rate 1/min | 0.2 - 1.9 | 1-2 |
| Sweep Flow Rate (no Vacuum) scfm | 0.02 - 0.2 | Not applicable as the module was not used for degassing |
| Sweep Flow Rate with Vacuum scfm | 0.004 - 0.04 | Not applicable as the module was not used for degassing |
| Typical Gas Flow Rate scfm | 0.004 - 0.4 | Not applicable as the module was not used for adding gas to a fluid |

During the process the tube side of the PermSelect PDMSXA 2.1m² module was connected to water at a temperature of 46 °C running at 1 L/min (cf. Table 5) thus representing the heating fluid while the shell side was connected to room temperature water at a temperature of 20 °C thus representing the cell culture process fluid to be warmed (cf. also Fig. 4).

**Table 5 Process conditions**

| | |
|---|---|
| Process Flow (L/m) | 1.0 |
| HW flow (L/m) | 1.0 |
| Mean Process in (°C) | 20.3 |
| Mean Process out (°C) | 40.4 |
| Mean HWS (°C) | 46.2 |
| Mean HWR (°C) | 25.1 |
| LMTD (°C) | 5.3 |
| Heat Transfer Rate Q (watt) | 1403 |
| Heat transfer coefficient U (w/m^2-k) | 127 |

To a skilled person it is clear, that this experimental configuration could be reversed. However, in this example, during operation the heating solution entered the hollow fibers of the PermSelect PDMSXA 2.1m² module, flowed through the hollow-fibers and exited the PermSelect PDMSXA 2.1m² module, in a sterile fashion while the process fluid to be warmed flowed over the outside of the hollow fibers (i.e. the shell side) . Thermocouple reading at the outlet of the shell side, i.e., the heated cell culture process fluid reached 40 °C - from 20.2 °C - in 50 seconds, which is approximately the residence time of the holdup volume. The results hence demonstrated that the heating up of the process solution by 20°C was almost instantaneous. This was much better than expected as it was first assumed that a recirculation of the cell culture process fluid to be warmed through the single-use dense silicone membrane hollow fiber module or passing the cell culture process fluid to be warmed through a second single-use dense silicone membrane hollow fiber module would be required. This assumption was based on the fact that it took 2-3 hours to heat up cell culture process fluid from 2-8°C to room temperature (i.e. by approximately 20°C) and then approximately another 3 hours to reach 37 °C using conventional methods such as heating blankets with bottles or 2D pillow bags. In another test. with the same set-up. with a heating solution flow of 2 L/min at 42°C. it was possible to heat a cold process solution flow at 2 L/min from 6°C to 33°C. These results prove the efficacy of this dense membrane hollow fiber module as an highly efficient heat exchanger.

Hence using a single-use dense membrane hollow fiber module for heating a cell culture process fluid can potentially enable single pass heating as well as for gassing or degassing. Moreover, it provides more operational flexibility compared to the devices used in the art as the single-use dense silicone membrane hollow fiber module used in this example is very compact. with only 3.5" diameter and 5.6" in length. and weighs less than half kg. compared to (20" x 29" x 27") and 150 kg of the DHX.

## Claims

1. A method for improving a cell culture comprising
a) heating a cell culture process fluid using a single-use dense membrane hollow fiber module (1) wherein said single-use dense membrane hollow fiber module comprises a plurality of dense membranes arranged as hollow fibers (2) in a housing (3)
and/or
b) wherein the cell culture has at least one cell culture chamber and at least the bottom or at least the top of said cell culture chamber (7) is covered with an insulation.

2. The method according to claim 1 wherein the singe-use dense membrane hollow fiber module comprises a tube side and a shell side and during the heating of the cell culture process fluid using said single-use dense membrane hollow fiber module (1) a heating fluid is passed through the tube side of said single-use dense membrane hollow fiber module (1) while the cell culture process fluid to be warmed is passed through the shell side of said single-use dense membrane hollow fiber module (1) or vice versa.

3. The method according to claim 1 or 2, wherein cell culture process fluid is selected from the group consisting of a cell culture medium a coating fluid employed to coat a cell culture chamber before culture of adherent cells in said chamber a washing fluid or a fluid to remove the cells from the cell culture.

4. The method according to claim 1 or claim 2, wherein the heating is carried out via passing the cell culture process fluid through single-use dense membrane hollow fiber module with a hold up volume of 0.2 liters.

5. The method according to claim 1 or claim 2, wherein the heating warms cell culture process fluid from between 20-22 °C to 35-42°C.

6. The method according to claim 1 or claim 2,wherein the heating takes place in the range of between 10 sec to 3 min.

7. The method according to claim 1 or claim 2 wherein the single-use dense hollow fiber module is a single-use dense silicone hollow fiber module

8. The method according to claim 1 wherein the cell culture is a stacked cell culture and at least the top or the bottom cell culture chamber are insulated.

9. The use of a dense membrane hollow fiber module as single use heat exchanger.
